(19) Europäisches Patentamt
European Patent Office
Office européen des brevets

(11) Veröffentlichungsnummer: **0 084 133**
**B1**

(12) # EUROPÄISCHE PATENTSCHRIFT

(45) Veröffentlichungstag der Patentschrift: **09.10.85**

(21) Anmeldenummer: **82111704.1**

(22) Anmeldetag: **16.12.82**

(51) Int. Cl.⁴: **C 07 C 29/04**, C 07 C 67/04, C 07 C 33/025, C 07 C 69/145

(54) **Verfahren zur Herstellung von ungesättigten Alkoholen und/oder deren Estern.**

(30) Priorität: **15.01.82 DE 3200990**

(43) Veröffentlichungstag der Anmeldung: **27.07.83 Patentblatt 83/30**

(45) Bekanntmachung des Hinweises auf die Patenterteilung: **09.10.85 Patentblatt 85/41**

(84) Benannte Vertragsstaaten: **CH DE FR GB LI NL**

(56) Entgegenhaltungen:
**EP-A- 0 001 325**
**DE-A- 2 755 945**
**GB-A- 1 283 517**
**BERICHTE DER DEUTSCHEN CHEMISCHEN GESELLSCHAFT, B and 76, Nr. 8, 1943**

(73) Patentinhaber: **BASF Aktiengesellschaft**
**Carl-Bosch-Strasse 38**
**D-6700 Ludwigshafen (DE)**

(72) Erfinder: **Rebafka, Walter, Dr.**
**Lessingstrasse 4**
**D-6945 Hirschberg (DE)**
Erfinder: **Nickels, Helmut, Dr.**
**Ginsterstrasse 15**
**D-6704 Mutterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 084 133 B1

Jouve, 18, rue St-Denis, 75001 Paris, France

**Beschreibung**

Die Erfindung betrifft ein verbessertes Verfahren zur Herstellung von ungesättigten Alkoholen und/oder deren Estern durch Umsetzen konjugierter Diene mit Wasser bzw. wäßrigen Carbonsäurelösungen in Gegenwart von sauren Ionenaustauschern.

Ungesättigte Alkohole und deren Ester sind begehrte Zwischenprodukte, die beispielsweise bei der Synthese von Naturprodukten wie den Terpenen eine bedeutende Rolle spielen. Es hat daher nicht an Versuchen gefehlt, vorteilhafte Verfahren zu deren Herstellung zu finden. Verfahren, die die Wasseranlagerung an Verbindungen mit isolierter Doppelbindung betreffen, sind in der Literatur zahlreich beschrieben. Die Wasseranlagerung erfolgt hierbei über das tertiäre Kation 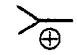 relativ schnell (günstige Gleichgewichtslage), kann sowohl in der Gasphase als auch in der flüssigen Phase (homogen oder zweiphasig), in Gegenwart von wäßrigen Mineralsäuren als auch mit Säuren auf festen Trägern, wie Ionenaustauschern durchgeführt werden ; die Selektivität ist im allgemeinen recht hoch ; Ionenaustauscher werden im allgemeinen nur aus verfahrenstechnischen Gründen verwendet. Genannt sei beispielsweise das Verfahren der DE-OS 2 755 945, gemäß dem man durch Wasseranlagerung an Citronellol in Alkanolen als Lösungsmittel und in Gegenwart von stark sauren Kationenaustauschern Hydroxycitronellol herstellen kann.

Ganz anders ist die Situation bei der Wasseranlagerung an Verbindungen mit konjugierten Dienen, wie sie gemäß der vorliegenden Anmeldung beschrieben wird.

Die Wasseranlagerung an konjugierte Diene verläuft über das extrem reaktionsfreudige Allylkation 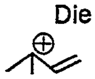 . Die große Reaktivität des Allylkations führt zu sehr großen Problemen bezüglich der Selektivität. Die Reaktion ist sehr unselektiv in der Gasphase und ebenfalls bei zweiphasiger Versuchsführung. Wäßrige Mineralsäuren bzw. organische Säuren sind nicht als Katalysatoren geeignet. So ist beispielsweise die Addition von Carbonsäuren an konjugierte Diene in Gegenwart von Schwefelsäure (vgl. Ber. *76*, (1943), 831) und Katalysatoren wie $PdCl_2/CuCl_2$ (vgl. Bull. Jap. Pet. Inst. *13*, (1971) 73, Austr. J. Chem. *1571*, (1971), 24) untersucht worden. Die Reaktionen sind jedoch sehr unspezifisch.

Weiterhin ist bekannt, 2-Methyl-3-buten-2-ol dadurch herzustellem, daß man Chlorwasserstoff an Isopren anlagert und das Reaktionsprodukt im alkalischen Milieu (vgl. DE-OS 30 21 414) oder neutralem Milieu (vgl. DE-AS 23 19 761) hydrolysiert.

Nachteilig bei diesen Verfahren sind der große Salzanfall, die Mehrstufigkeit sowie ausgeprägte Korrosionsprobleme.

Es ist weiterhin bekannt, 2-Methyl-3-buten-2-ol durch Reaktion von Acetylen mit Aceton und anschließende partielle Hydrierung herzustellen (vgl. M. De Malde, Chim. e Ind. (Milan) *45* (1963), S. 665 f). Acetylen ist jedoch teuer und technisch nicht einfach zu handhaben.

Außerdem ist die zweiphasige Umsetzung von Isopren mit Wasser in Tetrahydrofuran (THF) in Gegenwart des gelförmigen Ionenaustauschers Amberlite[R] IR 120 beschrieben (vgl. Bull. Inst. Chem. Res., Kyoto Univ. *1972*, 50 (4), S. 363-7). Wie aus Tabelle 1 dieser Arbeit zu sehen ist, werden in Abwesenheit eines Lösungsmittels nur dann gute Ausbeuten an dem gewünschten Alkohol II erhalten, wenn man mit einem — für die technische Herstellung absolut undiskutablen — Umsatz von nur 2 bis maximal 5 % vorliebnimmt. Bei höheren Temperaturen und damit höheren Ausbeuten wird die Umsetzung unselektiv.

Beim Arbeiten in Gegenwart von Tetrahydrofuran als Lösungsmittel verläuft die Reaktion zwar selektiver, doch lassen auch hierbei die erzielten Umsätze sehr zu wünschen übrig.

Es war daher die Aufgabe der Erfindung, das Verfahren zur Herstellung der definitionsgemäßen Alkohole und Ester durch Umsetzen von konjugierten Dienen mit Wasser bzw. mit wäßrigen Carbonsäurelösungen so zu verbessern, daß man auf einfache Weise und mit guten Umsätzen in einem Einstufenverfahren zu den gewünschten Verbindungen gelangt.

Es wurde nun überraschenderweise gefunden, daß die genannte Umsetzung bei niedrigeren Temperaturen, mit kürzeren Reaktionszeiten, mit erheblich höherem Umsatz und höherer Selektivität als bei den bekannten Verfahren verläuft, wenn man sie in homogener oder quasi-homogener Phase mit dazu geeigneten Lösungsmitteln in Gegenwart eines makroporösen sauren Ionenaustauschers vornimmt.

Gegenstand der Erfindung ist dementsprechend ein Verfahren zur Herstellung von ungesättigten Alkoholen und/oder deren Estern durch Umsetzen konjugierter Diene mit Wasser bzw. wäßrigen Carbonsäurelösungen in Gegenwart von sauren Ionenaustauschern sowie gegebenenfalls von Lösungsmitteln, das dadurch gekennzeichnet ist, daß man ein Dien der allgemeinen Formel I

$$\underset{R^1}{CH}=\underset{R^2}{C}-CH=\underset{R^3}{CH} \qquad (I)$$

in der

$R^1$, $R^2$ und $R^3$ für Wasserstoff oder eine Methylgruppe stehen,

$R^2$ darüber hinaus einen aliphatischen Kohlenwasserstoffrest mit 2 bis 6 C-Atomen, der eine nicht

konjugiert zu den Doppelbindungen des Diens stehende Doppelbindung enthalten kann, bedeuten kann und

$R^1$ und $R^3$ zusammen für die Gruppen $—CH_2—$ oder $—CH_2—CH_2—$ stehen können, ggf. in einem polaren aprotischen Lösungsmittel, das sich unter den Reaktionsbedingungen gegen alle Reaktionspartner inert oder weitgehend inert verhält,
in homogener oder quasi-homogener Phase in Gegenwart eines makroporösen sauren Ionenaustauschers umsetzt.

Besonders vorteilhaft gestaltet sich das erfindungsgemäße Verfahren, wenn man im Falle der Addition von Wasser Aceton, Dioxan oder ein cyclisches oder nichtcyclisches Sulfon, insbesondere Sulfolan, als Lösungsmittel verwendet und wenn man sowohl die Wasser- als auch die Säureaddition in Gegenwart von Lewatit® SPC 118, Lewatit SP 112 oder Amberlyst® XN 1010 als makroporösem sauren Ionenaustauscher vornimmt.

Geeignete konjugierte Diene sind beispielsweise Buta-1,3-dien, Isopren, Cyclopentadien, Cyclohexa-1,3-dien, Myrcen (2-Vinyl-5-methylhexa-1,4-dien) und insbesondere Isopren. Als Lösungsmittel sind polare aprotische Lösungsmittel geeignet, die mit dem Dien und dem Wasser bzw. der wäßrigen Carbonsäurelösung unter den Reaktionsbedingungen eine homogene oder, unter Zusatz eines Lösungsvermittlers, eine quasi-homogene Phase zu bilden vermögen. Bevorzugt sind naturgemäß einphasige echte Lösungen wie man sie beispielsweise mit Aceton, Dioxan, Tetrahydrofuran sowie cyclischen oder nichtcyclischen Sulfonen wie Sulfolan, Sulfolen, Dimethylsulfon und Sulfonal und Gemischen dieser Flüssigkeiten erhält. Mit besonderem Vorteil verwendet man Lösungsmittel, die höher als der entstehende Alkohol bzw. deren Ester sieden, da sich in diesem Falle die Aufarbeitung des Reaktionsgemisches wirtschaftlicher gestaltet. Solche Lösungsmittel sind beispielsweise die Sulfone, insbesondere Sulfolan. Die Lösungsmittelkonzentration liegt vorteilhaft zwischen 50 und 90 Vol.%, insbesondere zwischen 60 und 70 Vol.%.

Da es nach den bisherigen Beobachtungen darauf ankommt, daß die Reaktion des Diens mit dem Wasser bzw. der wäßrigen Carbonsäure an dem Ionenaustauscher in homogener oder quasi-homogener Phase stattfindet, ist es nicht erforderlich, daß die gesamte Flüssigkeit einphasig ist. Vielmehr genügt es, wenn man ein begrenzt wasserlösliches Lösungsmittel verwendet, da man auch in diesem Falle neben einer zweiten Phase eine Dien/Wasser/Lösungsmittel-Phase erhält. Hierfür geeignete Lösungsmittel sind beispielsweise Diethylether oder Ethylmethylketon.

Statt in einer echt homogenen, molekulardispersen Phase kann man die Reaktion auch in einer quasi-homogenen Phase vornehmen, d. h. einer Phase, die sich wegen ihres Dispersionsgrades wie eine homogene Lösung verhält. Hierdurch erweitert sich der Kreis der geeigneten Lösungsmittel um solche, die sich nicht oder nur begrenzt in Wasser lösen. In diesem Falle, etwa bei Verwendung höherer Ether wie Methyl-tert.-butylether, verwendet man in an sich bekannter Weise eine inerte oberflächenaktive Verbindung als Lösungsvermittler mit.

Daß sich die Lösungsmittel allgemein unter den Reaktionsbedingungen gegen alle Reaktionspartner inert oder weitgehend inert verhalten sollen, versteht sich hierbei von selbst.

Im Falle der Säureaddition wirkt die Säure meistens selber bereits als Lösungsmittel, mit dessen Hilfe sich eine homogene Phase ausbildet.

Das Molverhältnis von Wasser zu Dien liegt mit Vorteil zwischen 5 und 25, insbesondere zwischen 15 und 20. Mit steigendem Molverhältnis steigt auch der Dienumwandlungsgrad.

Für die Herstellung von Estern verwendet man wäßrige Carbonsäurelösungen. Es werden bevorzugt Lösungen von niederen aliphatischen Carbonsäuren wie Essigsäure und Propionsäure eingesetzt. Die Säurekonzentration in den wäßrigen, das Lösungsmittel enthaltenden Lösungen liegt mit Vorteil zwischen 50 und 90 Vol.%, insbesondere zwischen 70 und 80 Vol.%.

Die erfindungsgemäß zu verwendenden makroporösen sauren Ionenaustauscher sind vornehmlich sulfonierte vernetzte Copolymerisate auf der Basis von Styrol und Divinylbenzol (Gerüstsubstanz, Matrix), die aufgrund spezieller Herstellungsverfahren eine makroporöse Struktur ähnlich derjenigen der klassischen Adsorbtion $Al_2O_3$, $SiO_2$ oder Aktivkohle aufweisen. Diese Porenstruktur ist im wesentlichen konstant, also unabhängig vom Reaktionsmedium und den dadurch bedingten Quellvorgängen, wodurch sich die makroporösen Harze von den länger bekannten Austauschern mit gelartigen Eigenschaften unterscheiden. Die erfindungsgemäß zu verwendenden Harze haben einen mittleren Porendurchmesser von mehr als 50 Angström und eine nach Brunauer, Emmet und Teller bestimmte innere Oberfläche (« BET »-Oberfläche), die größer ist als 1 $m^2/g$.

Näheres zu den Eigenschaften und Bestimmungsmethoden der makroporösen Ionenaustauscher ist der Firmenschrift von A.R. Pitochelli, « Ion Exchange Catalysis and Matrix Effects » (veröffentlicht nach 1974) der Firma Rohm and Haas, Philadelphia zu entnehmen. Als weiteres Schrifttum seien die Arbeiten von R. Kunin « Amber-hi-lites ; Catalysis with Ion Exchange Resins », 1972, und von R. Kunin und R. Hetherington, « A Progress Report on the Removal of Colloids from Water by Macroreticular Ion Exchange Resins », 1969, beide ebenfalls von Rohm and Haas, genannt.

Geeignete makroporöse saure Ionenaustauscher sind seit längerem im Handel erhältlich. Genannt seien beispielsweise Lewatit SPC 118 und SP 112, Amberlyst 15, XN-1005 und XN 1010, Dowex® HGR und MSC-1 und Kastel® C 350 P und C 386. Die Kationenaustauscher werden in der handelsüblichen $H^{\oplus}$-Form eingesetzt.

Die optimale Temperatur für die erfindungsgemäße Umsetzung hängt stark von dem eingesetzten Dien ab. So liegt beispielsweise die optimale Temperatur für die Hydratisierung von Isopren zwischen 35 und 45 °C, die für Butadien zwischen 80 und 110 °C. Im allgemeinen liegen die Reaktionstemperaturen je nach dem Dien zwischen 0 und 150 °C, insbesondere zwischen 20 und 120 °C.

Die Umsetzung erfolgt im allgemeinen unter dem Dampfdruck des Diens bei der entsprechenden Reaktionstemperatur.

Die Reaktionszeit beträgt bei Anwendung der vorteilhaften Temperaturbereiche im allgemeinen 0,2 bis 10 Stunden, insbesondere 2 bis 6 Stunden bei diskontinuierlicher Arbeitsweise.

Die Umsätze liegen je nach eingesetztem Dien zwischen 40 und 45 % bei Alkohol- bzw. Ester-Gesamtselektivitäten zwischen 80 und 85 %.

Die Wasser- bzw. Säureanlagerung kann im Falle eines unsymmetrischen Diens, beispielsweise des Isoprens, formal unter 1,2-, 3,4- und 1,4-Addition erfolgen, wobei die Hydroxylgruppe bzw. die Estergruppierung in 1-, 2-, 3- und 4-Stellung eintreten kann. Diese Möglichkeiten werden jedoch entsprechend bekannten Gesetzmäßigkeiten insofern eingeschränkt, als bevorzugt tertiäre und danach sekundäre Alkohole bzw. Ester vor den primären Alkoholen bzw. Estern gebildet werden. Wegen der Reaktionsfähigkeit des konjugierten Systems kommt die Reaktion nach der Monoaddition zunächst zum Stillstand, so daß man es wie üblich in der Hand hat, Bis-Additionen weitgehend zu unterdrücken. Aufgrund der genannten Gesetzmäßigkeiten erhält man eines der möglichen Verfahrensprodukte in aller Regel in hohem Überschuß. Da meist jedoch alle entstehenden Alkohole bzw. Ester wertvolle Verbindungen sind, ist es sinnvoll, die einzelnen Selektivitäten (Ausbeute, bezogen auf Umsatz) zur Veranschaulichung des Verfahrenserfolges zu einer Gesamtselektivität zu addieren.

Nichtumgesetztes Dien kann von dem Reaktionsgemisch durch Destillation wieder abgetrennt und wiederverwendet werden. Aus dem wäßrigen dienfreien Reaktionsgemisch lassen sich die gebildeten Alkohole — bzw. Alkohole und Ester — in an sich bekannter Weise und üblicher Weise isolieren. Auch das Wasser/Lösungsmittelgemisch kann bei der Umsetzung erneut verwendet werden.

Mit Hilfe des erfindungsgemäßen Verfahrens, das mit besonderem Vorteil unschwer auch kontinuierlich gestaltet werden kann, können die als Zwischenprodukte begehrten ungesättigten Alkohole und/oder deren Ester auf einfache und umweltfreundliche Weise mit guten Umsätzen und guten Selektivitäten hergestellt werden.

## Beispiel 1

In einem mit Rührer ausgestatteten Autoklaven wurde eine homogene Lösung aus 56 g (0,8 mol) Isopren und 1 000 ml einer 65 vol.%igen wäßrigen Sulfolanlösung in Gegenwart von 50 g des Kationenaustauschers Lewatit SPC 118 bei 40 °C umgesetzt. Bei dieser Umsetzung betrug das Molverhältnis von Wasser zu Isopren 24 : 1. Nach 4-stündigem Rühren bei 40 °C betrug der Umsatz 40 %. Die Selektivität an 2-Methyl-but-3-en-2-ol betrug 73 %, die an 3-Methyl-but-2-en-1-ol (Prenol) 10 %.

## Beispiel 2

Analog Beispiel 1 wurde eine homogene Lösung aus 6,2 g (0,11 mol) Butadien und 100 ml einer 65 vol.%igen wäßrigen Sulfolanlösung in Gegenwart von 5 g Lewatit SPC 118 bei 100 °C umgesetzt. Nach 6-stündigem Rühren bei 100 °C betrug der Umsatz 76 %. Man erhielt But-1-en-3-ol in einer Selektivität von 60 % und But-2-en-1-ol (Crotylalkohol) in einer Selektivität von 26 %.

## Beispiel 3

Analog Beispiel 1 wurde eine homogene Lösung aus 5,6 g (0,08 mol) Isopren und 100 ml einer 75 vol.%igen wäßrigen Essigsäurelösung in Gegenwart von 5 g Lewatit SPC 118 bei 40 °C umgesetzt. (Molverhältnis Essigsäure : Isopren = 15,6 ; Molverhältnis Wasser : Isopren = 17,3). Nach 6-stündigem Rühren bei 40 °C betrug der Umsatz 73 %. Es wurden 2-Methyl-but-3-en-2-ol in einer Selektivität von 48 %, Prenol mit einer Selektivität von 2 % und Prenylacetat mit einer Selektivität von 30 % erhalten.

## Beispiel 4

Analog Beispiel 1 wurde eine Lösung aus 5,6 g (0,08 mol) Isopren und 100 ml einer 65 vol.%igen wäßrigen Propionsäure (Molverhältnisse : Wasser : Isopren = 24,3 ; Propionsäure zu Isopren = 10,7) in Gegenwart von 5 g Lewatit SPC 118 bei 35 °C umgesetzt. Nach 6-stündigem Rühren bei 35 °C betrug der Umsatz 20 %. Es wurden 2-Methyl-but-3-en-2-ol in einer Selektivität von 57 % und Prenylpropionat in einer Selektivität von 29 % erhalten.

## Beispiel 5

a) Analog Beispiel 1 wurde eine Lösung aus 6,2 g (0,11 mol) Butadien mit 100 ml einer 80 vol.%igen wäßrigen Essigsäurelösung in Gegenwart von 5 g Lewatit SPC 118 bei 80 °C umgesetzt. Nach 6-

stündigem Rühren bei 80 °C betrug der Umsatz 48 % bei folgender Produktverteilung :

But-1-en-3-ol (13 %)
But-1-en-3-ol-acetat (30 %)
Crotylalkohol (9 %)
Crotylacetat (40 %)
Gesamtselektivität : 92 %

b) Vergleichsbeispiel

Es wurde wie unter a) beschrieben gearbeitet, jedoch wurde anstelle der wäßrigen Essigsäure reine Essigsäure verwendet. Nach 6-stündigem Rühren bei 80 °C betrug der Umsatz an Butadien 94 %, die Gesamtselektivität jedoch nur 37 %.

Beispiel 6 bis 8 und Vergleichsbeispiele

Analog Beispiel 1 wurde jeweils eine homogene Lösung aus 65 Vol.% aus Sulfolan, 10 Vol.% Isopren und 25 Vol.% Wasser mit 5 g eines Ionenaustauschers versetzt und anschließend 4 Stunden unter Rühren auf 40 °C erwärmt. Die hierbei erzielten Umsätze an Isopren sowie die Selektivitäten an 2-Methyl-but-3-en-2-ol und Prenol sind in der folgenden Tabelle angegeben.

| Beispiel | Ionenaustauscher makroporös | Umsatz [%] | Selektivität Methyl-butenol [%] | Prenol [%] |
|---|---|---|---|---|
| erfindungsgemäß | | | | |
| 6 | Lewatit SPC 118 | 40 | 73 | 12 |
| 7 | Lewatit SP 112 | 25 | 59 | 12 |
| 8 | Amberlyst XN 1010 | 23 | 67 | 9 |
| zum Vergleich | gelförmig | | | |
| 9 | Amberlite JR 120 | 7 | 64 | 9 |
| 10 | Amberlite XE 365 A | <5 | nicht bestimmt | |
| 11 | Nafion® | <5 | nicht bestimmt | |
| 12 | Dowex 50 WX | <5 | nicht bestimmt | |
| 13 | Lewatit S 100 | <5 | nicht bestimmt | |
| 14 | Dowex HER W2 | <5 | nicht bestimmt | |

**Patentansprüche**

1. Verfahren zur Herstellung von ungesättigten Alkoholen und/oder deren Estern durch Umsetzen konjugierter Diene mit Wasser bzw. wäßrigen Carbonsäurelösungen in Gegenwart von sauren Ionenaustauschern sowie gegebenenfalls von Lösungsmitteln, dadurch gekennzeichnet, daß man ein Dien der allgemeinen Formel I

$$\underset{R^1}{CH}=\underset{R^2}{C}-CH=\underset{R^3}{CH} \qquad (I)$$

in der

$R^1$, $R^2$ und $R^3$ für Wasserstoff oder eine Methylgruppe stehen,

$R^2$ darüber hinaus einen aliphatischen Kohlenwasserstoffrest mit 2 bis 6 C-Atomen, der eine nicht konjugiert zu den Doppelbindungen des Diens stehende Doppelbindung enthalten kann, bedeuten kann und

$R^1$ und $R^3$ zusammen für die Gruppen $-CH_2-$ oder $-CH_2-CH_2$ stehen können, ggf. in einem polaren aprotischen Lösungsmittel, das sich unter den Reaktionsbedingungen gegen alle Reaktionspartner inert oder weitgehend inert verhält,

in homogener oder quasi-homogener Phase in Gegenwart eines makroporösen sauren Ionenaustauschers umsetzt.

2. Verfahren nach Anspruch 1, dadurch gekennzeichnet, daß man im Falle der Addition von Wasser an die Diene I als Lösungsmittel Aceton, Dioxan oder ein cyclisches oder nichtcyclisches Sulfon oder Gemische dieser Lösungsmittel verwendet.

3. Verfahren nach Anspruch 2, dadurch gekennzeichnet, daß man Sulfolan als Lösungsmittel verwendet.

4. Verfahren nach den Ansprüchen 1 bis 3, dadurch gekennzeichnet, daß der makroporöse saure Ionenaustauscher einen mittleren Porendurchmesser aufweist, der größer als 50 Angström ist.

5. Verfahren nach den Ansprüchen 1 bis 4, dadurch gekennzeichnet, daß man Lewatit® SPC 118, Lewatit SP 112 oder Amberlyst® XN 1010 als makroporösen sauren Ionenaustauscher verwendet.

**Claims**

1. A process for the preparation of unsaturated alcohols and/or esters thereof by reacting conjugated dienes with water or aqueous carboxylic acid solutions in the presence of acid ion exchangers and optional solvents, wherein a diene of the general formula I

$$\underset{R^1}{CH}=\underset{R^2}{C}-CH=\underset{R^3}{CH} \qquad (I)$$

where

$R^1$, $R^2$ and $R^3$ each denote hydrogen or methyl, it being possible for

$R^2$ to also denote an aliphatic hydrocarbon radical of 2 to 6 carbon atoms which may contain a double bond which is not in a conjugated position relative to the double bonds of the diene, and

$R^1$ and $R^3$ together can denote $-CH_2-$ or $-CH_2-CH_2-$, is reacted in a homogeneous or quasi-homogeneous phase in the presence of a macroporous acid ion exchanger and in the presence or absence of a polar aprotic solvent which is inert or substantially inert to all reactants under the reaction conditions.

2. A process as claimed in claim 1, wherein, when water is added on to the diene I, acetone, dioxane or a cyclic or non-cyclic sulfone or a mixture of these solvents is used as solvent.

3. A process as claimed in claim 2, wherein sulfolane is used as solvent.

4. A process as claimed in claims 1 to 3, wherein the macroporous acid ion exchanger has a mean pore diameter of more than 50 Å.

5. A process as claimed in claims 1 to 4, wherein Lewatit® SPC 118, Lewatit SP 112 or Amberlyst® XN 1010 is used as the macroporous acid ion exchanger.

**Revendications**

1. Procédé pour la préparation d'alcools non saturés et/ou de leurs esters, par réaction de diènes conjugués avec l'eau ou des solutions aqueuses d'acides carboxyliques en présence d'échangeurs d'ions acides et, le cas échéant, de solvants, caractérisé en ce qu'on fait réagir un diène de formule générale I

$$\underset{R^1}{CH}=\underset{R^2}{C}-CH=\underset{R^3}{CH} \qquad (I)$$

dans laquelle

$R^1$, $R^2$ et $R^3$ sont mis chacun pour un atome d'hydrogène ou un groupe méthyle,

$R^2$ peut représenter en outre un radical hydrocarboné aliphatique à 2-6 atomes de carbone, qui peut contenir une double liaison non conjuguée avec les doubles liaisons du diène et

$R^1$ et $R^3$ peuvent constituer ensemble les groupes $-CH_2-$ ou $-CH_2-CH_2-$

le cas échéant dans un solvant aprotique polaire qui a un comportement inerte ou largement inerte à l'égard de tous les partenaires réactionnels dans les conditions de la réaction, en phase homogène ou quasi homogène et en présence d'un échangeur d'ions acide macroporeux.

2. Procédé selon la revendication 1, caractérisé en ce que dans le cas de l'addition d'eau aux diènes I, on utilise comme solvant l'acétone, le dioxanne, un sulfone cyclique ou non cyclique ou des mélanges de ces solvants.

3. Procédé selon la revendication 2, caractérisé en ce qu'on utilise comme solvant le Sulfolane.

4. Procédé selon l'une quelconque des revendications 1 à 3, caractérisé en ce que l'échangeur d'ions acide macroporeux présente un diamètre moyen de pores qui est supérieur à 50 angströms.

5. Procédé selon l'une quelconque des revendications 1 à 4, caractérisé en ce qu'on utilise comme échangeur d'ions acide macroporeux le Lewatit® SPC 118, le Lewatit SP 112 ou l'Amberlyst® XN 1010.